# EUROPEAN PATENT APPLICATION

(11) **EP 3 957 236 A1**
(43) Date of publication of application: **23.02.2022**
(21) Application number: 20192050.1
(22) Date of filing: 21.08.2020
(51) Int. Cl.: A61B 5/021, A61B 5/022, A61B 5/00

(54) **FAILURE DETECTION DEVICE AND METHOD FOR A BLOOD PRESSURE MONITORING DEVICE**

(71) Applicant: Koninklijke Philips N.V., 5656 AG Eindhoven (NL)
(72) Inventor: FRANCK, Christoph Florian, 5656 AE Eindhoven (NL)
(74) Representative: Philips Intellectual Property & Standards

(57) **Abstract**

The present invention relates to a failure detection device (25) and method for a blood pressure monitoring device (20, 20'). To avoid potentially harmful injuries of a subject, the failure detection device comprises a data input (251) configured to obtain a pressure signal and/or a gas flow signal, the pressure signal indicating a cuff pressure of a cuff (10) that is configured to be mounted to a subject's body part and to be inflated for performing a blood pressure measurement and the gas flow signal indicating gas flow entering the cuff during inflation and leaving the cuff during deflation; a signaling output (252) configured to output a failure signal; and a processor (253) configured to detect, based on the obtained pressure signal and/or the obtained gas flow signal, a deflation failure indicating a failure to deflate the cuff after a blood pressure measurement below a safety cuff pressure and to control the signaling output (252) to output a failure signal indicating a deflation failure in case a deflation failure is detected.

## Description

### FIELD OF THE INVENTION

The present invention relates to a failure detection device and method for a blood pressure monitoring device. The present invention relates further to a blood pressure monitoring device and a blood pressure monitoring system and a computer program.

### BACKGROUND OF THE INVENTION

Non-invasive blood pressure (NiBP) measurement refers to methods used to measure a patient's (or, more generally, a subject's) systolic and diastolic blood pressure that do not require inserting a catheter or pressure sensor into one of the patient's arteries. NiBP methods usually comprise applying pressure to one of the patient's extremities, often via an inflatable cuff wrapped around the extremity, and then either recording the Korotkoff sounds that occur if the cuff pressure is between the diastolic and the systolic pressure (ausculatory method or Riva-Rocci/RR-method) or detecting the small pressure oscillations in the cuff caused by the pulsatile blood flow (oscillometric method).

For automated NiBP measurement, the oscillometric method is more common, as it does not require a microphone to pick up sound at the patient's extremity. It requires an inflatable cuff, connected to the NiBP monitor (the blood pressure monitoring device) via an air hose, a means for inflating the cuff, a means for measuring the pressure inside the cuff, and a means for deflating the cuff.

The cuff is usually inflated by using a pump, the pressure is measured by a pressure sensor inside the NiBP monitor, and for safety reasons, a NiBP monitor usually contains several independent valves that, if at least one of the valves is opened, allow the air to exit the cuff.

For economic reasons, the cuff and the attached air hose are often passive components that have no electrical connections for sensors or valves. This means that the pressure inside the cuff is actually measured by a sensor in the NiBP monitor. In this common case, the cuff pressure can only be determined correctly if the pneumatic path (the air hose) between the cuff and the NiBP monitor is clear and there is no or only very little air flow (i.e. the valves are closed and the pump is not running or only delivering a small air flow).

NiBP measurement is considered safe and generally involves little risk for the patient. However, if automated NiBP measurements are taken while the patient is unconscious or otherwise helpless, and the NiBP cuff fails to deflate after a measurement, compartment syndrome and injury from prolonged lack of blood circulation is possible. This type of injury manifests gradually, over the course of many minutes or hours, and can be prevented entirely by removing the cuff from the extremity if the situation is detected in time.

Failure to deflate (also called deflation failure herein) can occur if the air hose is compressed, blocked or bent, and therefore does not necessarily imply that the automated NiBP monitor is defective. Patient endangerment or harm due to failure to deflate is rare, but some cases have been reported.

### SUMMARY OF THE INVENTION

It is an object of the present invention to enable a safe and fast detection of a deflation failure of a blood pressure monitoring device.

In a first aspect of the present invention a failure detection device for a blood pressure monitoring device is presented comprising:
- a data input configured to obtain a pressure signal and/or a gas flow signal, the pressure signal indicating a cuff pressure of a cuff that is configured to be mounted to a subject's body part and to be inflated for performing a blood pressure measurement and the gas flow signal indicating gas flow entering the cuff during inflation and leaving the cuff during deflation;
- a signaling output configured to output a failure signal; and
- a processor configured to detect, based on the obtained pressure signal and/or the obtained gas flow signal, a deflation failure indicating a failure to deflate the cuff after a blood pressure measurement below a safety cuff pressure and to control the signaling output to output a failure signal indicating a deflation failure in case a deflation failure is detected.

In a further aspect of the present invention a blood pressure monitoring device is presented comprising:
- a pressure generating unit configured to inflate a cuff that is configured to be mounted to a subject's body part;
- a valve configured to deflate the cuff;
- a pressure sensor configured to measure the cuff pressure;
- a processor configured to control the pressure generating unit and the valve and to determine the subject's blood pressure based on the measured cuff pressure; and
- a failure detection device as disclosed herein.

In a further aspect of the present invention a blood pressure monitoring system is presented comprising:
- a cuff configured to be mounted to a subject's body part; and
- a blood pressure monitoring device as disclosed herein, the blood pressure monitoring device being configured to control the cuff and non-invasively measure the subject's blood pressure.

In yet further aspects of the present invention, there are provided a corresponding failure detection method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, device, system, computer program and medium have similar and/or identical preferred embodiments as the claimed system, in particular as defined in the dependent claims and as disclosed herein.

The present invention is based on the idea that the risk to the patient due to possible failure to deflate the cuff can be mitigated if steps are taken to ascertain that the cuff was deflated to a harmless pressure after the measurement. A failure signal, such as an alarm, may thus be signalled if the cuff wrapped around a patient's extremity fails to deflate after a non-invasive blood pressure measurement. This risk of injuries of a patient can thus be mitigated.

The patient monitoring device records the amount of air entering and leaving the cuff during a measurement and/or monitors the cuff pressure signal. This information may then be used to detect events that indicate that the pneumatic path has become blocked, such as a sudden drop from a cuff pressure in the harmful range to the ambient pressure value, and that it may not be possible to deflate the cuff. If any such condition is detected, it may be interpreted as a deflation failure so that e.g. an alert may be generated and issued to instruct the user to remove the cuff from the patient and check the air hose and cuff for possible blockages.

In this context, the safety cuff pressure may be understood as a cuff pressure that is not harmful to the patient. The safety cuff pressure may be set in advance, either as general value valid for all patients or as category-related value selected from different values for different categories (or groups) of patients (e.g. depending on features of patient, such as age, gender, weight, height, health status, etc.) or even as patient-individual value set for the individual patient.

There are multiple options to detect a deflation failure. According to an embodiment of the failure detection device the processor is configured to
- detect a deflation failure based on the obtained cuff pressure signal,
- control a valve that is configured to deflate the cuff to intermittently deflate the cuff after the blood pressure measurement,
- control a pressure sensor that is configured to measure the cuff pressure to measure the remaining cuff pressure in between the periods of deflation, and
- detect a deflation failure based on the intermittently measured remaining cuff pressure values.

In this way a detection failure can be reliably detected without requiring any additional hardware in the patient monitoring device beyond the hardware that is conventionally available in a patient monitoring device.

Hereby, the processor may particularly be configured to check, for the intermittently measured remaining cuff pressure values, if the remaining cuff pressure is below the safety cuff pressure.

In particular, it may be checked if an intermittently measured remaining cuff pressure value is below the safety cuff pressure and below a second pressure threshold corresponding to ambient pressure or unpressurized state of the cuff and to detect it as deflation failure if the previous intermittently measured pressure value was above the safety cuff pressure and the current intermittently measured remaining cuff pressure value is below the safety cuff pressure and below the second pressure threshold. The processor is preferably configured to interrupt the deflation process after a blood pressure measurement by closing the deflation valves at a point where the cuff pressure is below the maximum safety cuff pressure and still above the ambient pressure range.

With this embodiment it can be ensured that, while the measurement is ongoing, an unexpected pressure drop from above the safe range into the ambient range shall immediately trigger the failure signal, e.g. a deflation failure alarm. During deflation after a measurement, the processor shall interrupt the deflation briefly, aiming to stop it in the safe pressure range, but above ambient pressure. Several stops may be required to achieve this, depending on how accurately the cuff pressure can be predicted while the deflation valve(s) are open. Once a pressure value in the safe range, but above ambient range was detected, the deflation can be finished without further interruptions.

Alternatively, it may be checked if an intermittently measured remaining cuff pressure value is below the safety cuff pressure and below a second pressure threshold corresponding to ambient pressure or unpressurized state of the cuff, in order to control a pressure generating unit to re-inflate the cuff if an intermittently measured remaining cuff pressure value is below the safety cuff pressure and below the second pressure threshold, and to detect it as deflation failure if the re-inflation was not successful. Hereby, success may be understood as reaching a higher cuff pressure that it within the expected range for the parameters (e.g. duration, pump power, etc.) of the inflation. A deflation failure would then be detected if either a higher pressure is not reached (if e.g. an air hose connecting the pressure generating unit (e.g. a pump) is disconnected from the cuff or has a leak), or if a much higher pressure than expected is observed (if e.g. the air hose is blocked and only a tiny volume of the air hose up to the blockage is inflated).

In another embodiment the processor is configured to detect a deflation failure based on the obtained gas flow signal, wherein the obtained gas flow signal indicates a first measured amount of gas entering the cuff during inflation and a second measured amount of gas leaving the cuff during deflation. In the course of one measurement, there may be multiple inflation and deflation phases. For example, the blood pressure monitor could inflate the cuff to a certain pressure, perform a few oscillation measurements, each followed by a small deflation, and then detect that the measurement requires oscillation amplitude values for higher cuff pressures and inflate the cuff to a higher pressure.

Hereby, a difference value may be obtained by subtracting the second amount from the first amount and to control the signaling output to output a failure signal indicating a deflation failure if the difference value exceeds a residual value, wherein the residual value is preferably based on one or more of:
- size or maximum gas volume that can be pumped into the cuff;
- one or more features of the subject, the one or more features including age, gender, height, weight, and health status;
- maximum gas volume pumped into the cuff during blood pressure measurement;
- maximum cuff pressure during blood pressure measurement;
- a constant threshold; and
- a fraction of the air volume pumped into the cuff.

In an implementation, since larger cuff sizes may retain more residual air than smaller cuff sizes, the residual value should be higher for larger cuffs to avoid false alarms.

In another implementation, since usually smaller cuff sizes are rather used for neonatal and pediatric patients than for adults, it is possible to determine the most likely range of cuff sizes from patient category.

In another implementation, the maximum gas volume pumped into the cuff and/or the maximum cuff pressure attained can be used to require that the higher the attained maximum pressure, the smaller the fraction of the maximum gas volume that is allowed to remain in the cuff. For example, if a maximum pressure of 100 mmHg was attained, it may be required that at least 90% of the maximum gas volume are vented from the cuff, while if a maximum pressure of 200 mmHg was attained, it could require that at least 95% of the maximum attained air volume are vented to avoid a detection of a deflation failure and e.g. triggering an alarm.

If the other criteria for determining the tolerated residual volume are not available or not used, a fixed threshold can be used in another implementation to avoid false alarms, e.g. if the residual volume is below x cm³, no deflation failure alarm is raised. Alternatively, the fixed threshold can be used if the other criteria would result in allowed residual values that are too small and therefore likely to trigger false alarms, e.g. if it is known that even with the smallest cuff size, x cm³ of residual volume will not result in a possibly harmful cuff pressure, then the alarm will not be raised if the residual volume is below x cm³ even if the threshold were lower than x according to the other criteria. Thus, a fallback option is available to avoid implausibly low allowed residual values.

According to another embodiment the signaling output is configured to output a failure signal indicating one or more of an instruction to remove the cuff from the subject's body part, an urgency of the deflation failure, and a strength of the deflation failure. The recipient of the failure signal thus knows if and how to act and how fast an action is required. Injuries of a patient can thus be avoided.

According to another embodiment the processor is configured to detect a deflation failure by detecting a sudden large change of the cuff pressure during inflation, in particular a change of the cuff pressure exceeding a change threshold within a predetermined period, and/or by detecting a deviation above a deviation threshold from a predicted cuff pressure that is predicted based on the obtained pressure signal and/or the obtained gas flow signal.

There are several failure modes that may be addressed. One failure mode refers to rupture of the air line and/or cuff. This will result in a rapid drop of the measured pressure down to ambient pressure. While this failure mode will most likely not result in the cuff retaining its current air pressure, the failure detection device and method can hardly ensure that deflation has actually happened. A failure signal should thus be emitted in this case as the state of the cuff is no longer known.

Another failure mode refers to sudden blockage of the air line, for example when it is compressed or bent. When the blood pressure monitor attempts to deflate the cuff with a blockage of the air hose in place, this causes a much larger pressure drop than expected (as only the tiny residual volume in the air hose is vented, and afterwards the pressure sensor only detected ambient pressure). When the blood pressure monitor attempts to inflate the cuff while the air hose is blocked, this results in an unexpectedly rapid increase in detected pressure, as only the tiny volume of the air hose up to the blockage is being inflated. In both cases (either a larger-than-expected pressure drop in response to a deflation attempt, or a larger-than-expected pressure increase in response to an inflation attempt), a failure signal should be emitted.

The blood pressure monitoring device according to the present invention comprises a pressure generating unit (e.g. a pump), a valve, a pressure sensor configured to measure the cuff pressure, a processor, and a failure detection device as described above. It may further comprise in an embodiment a flow meter configured to measure gas flow entering the cuff during inflation and leaving the cuff during deflation. The failure detection device and method may thus use, as input from the blood pressure monitoring device, the measured cuff pressure and, optionally, the measured gas flow.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a blood pressure monitoring system including a first embodiment of a blood pressure monitoring device according to the present invention;
Fig. 2 shows a schematic diagram of a blood pressure monitoring system including a second embodiment of a blood pressure monitoring device according to the present invention;
Fig. 3 shows a schematic diagram of an embodiment of a failure detection device according to the present invention;
Fig. 4 shows a flow chart of a first embodiment of a failure detection method according to the present invention;
Fig. 5 shows a flow chart of a second embodiment of a failure detection method according to the present invention; and
Fig. 6 shows a flow chart of a third embodiment of a failure detection method according to the present invention.

### DETAILED DESCRIPTION OF EMBODIMENTS

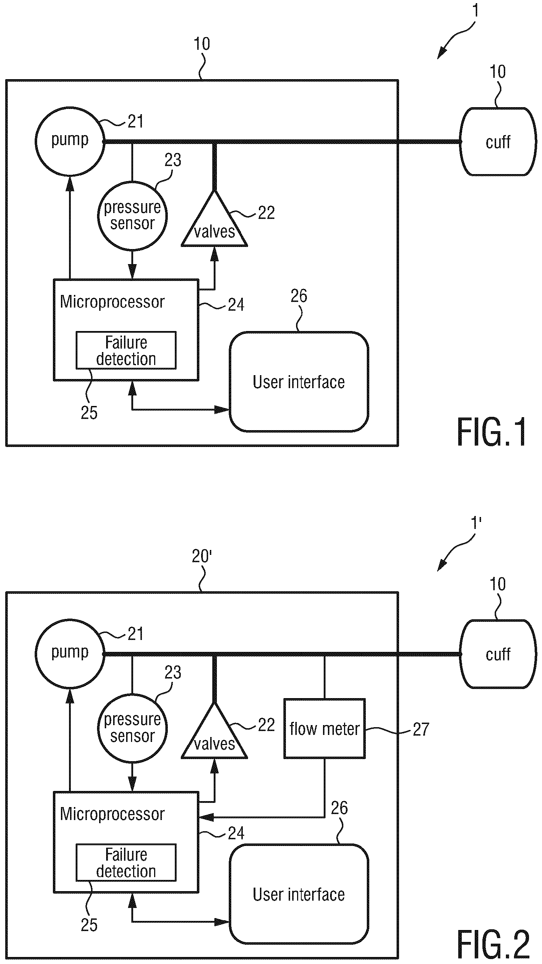

Fig. 1 shows a schematic diagram of a blood pressure monitoring system 1 including a first embodiment of a blood pressure monitoring device 20 according to the present invention. The blood pressure monitoring system 1 comprises a cuff 10 that is configured to be mounted to a subject's body part, e.g. around the upper part of the subject's arm, and a blood pressure monitoring device 20 that is configured to control the cuff 10 and non-invasively measure the subject's blood pressure.

The blood pressure monitoring device 20 comprises a pressure generating unit 21, e.g. a pump, that is configured to inflate the cuff 10, a valve 22 that is configured to deflate the cuff 10, a pressure sensor 23 that is configured to measure the cuff pressure, and a processor 24 configured to control the pressure generating unit 21 and the valve 22 and to determine the subject's blood pressure based on the measured cuff pressure. Further, a failure detection device 25 is provided that is configured to detect a deflation failure indicating a failure to deflate the cuff after a blood pressure measurement and to output a failure signal indicating a deflation failure in case a deflation failure is detected. A user interface 26 may be provided, e.g. comprising one or more of a display, keypad, loudspeaker and touchpad, to issue such a failure signal, e.g. in the form of a visible and/or audible alarm.

The failure detection device 25 may, as shown in Fig. 1, be part of the processor 24 that is programmed accordingly to carry out the functions of the failure detection device 25. In other embodiments, a separate processor or other hard- and/or software may be provided to implement the failure detection device 25. The failure detection device 25 may thus also be provided as a separate device external from the processor 24 or even external from the blood pressure measuring device 20.

Fig. 2 shows a schematic diagram of a blood pressure monitoring system 1' including a second embodiment of a blood pressure monitoring device 20' according to the present invention. In this embodiment, the blood pressure monitoring device 20' further comprises, in addition to the elements of the blood pressure monitoring device 20 shown in Fig. 1, a flow meter 27 that is configured to measure gas flow entering the cuff 10 during inflation and leaving the cuff 10 during deflation.

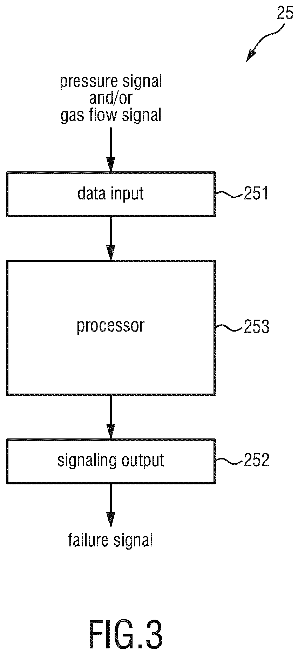

Fig. 3 shows a schematic diagram of an embodiment of a failure detection device 25 according to the present invention. It comprises a data input 251, a signaling output 252 and a processor 253.

The data input 251 is configured to obtain a pressure signal (from the pressure sensor 23) and/or a gas flow signal (from the flow meter 27). The pressure signal indicates a cuff pressure of the cuff 10 and the gas flow signal indicates gas flow entering the cuff 10 during inflation and leaving the cuff during deflation. The signaling output is configured to output a failure signal. The data input 251 and the signaling output 252 may be implemented in hard- and/or software. For instance, they may be implemented as data interfaces of the processor 253, for receiving and outputting the respective signals or data via signal lines or in a wireless manner (e.g. via Bluetooth) from the respective sensors.

The processor 253 is configured to detect, based on the obtained pressure signal and/or the obtained gas flow signal, a deflation failure indicating a failure to deflate the cuff after a blood pressure measurement below a safety cuff pressure and to control the signaling output 252 to output a failure signal indicating a deflation failure in case a deflation failure is detected. In an embodiment the failure signal may indicate one or more of an instruction to remove the cuff from the subject's body part, an urgency of the deflation failure, and a strength of the deflation failure. With this information a user (e.g. a caregiver) knows how to act to avoid or reduce injuries of the patient.

Embodiments how to detect a deflation failure will be described in more detail below.

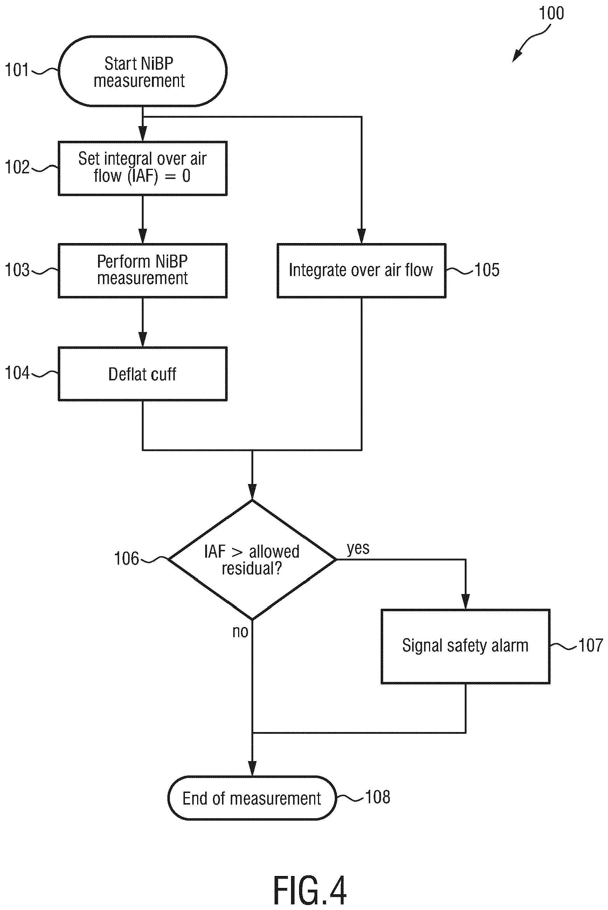

Fig. 4 shows a flow chart of a first embodiment of a failure detection method 100 according to the present invention, which may be carried out by a failure detection device 25 in a blood pressure monitoring device 20' shown in Fig. 2 since this direct method requires that a flow meter 27 is included in pneumatic path from the pump 21 to the cuff 10. As mentioned above, the flow meter 27 is used to measure the amount of gas (in particular air) entering the cuff 10 during the NiBP measurement, and the amount of gas leaving the cuff 10 when it is deflated after the measurement.

In step 101 the NIBP measurement is started, e.g. by a user instructing the blood pressure monitor to take a measurement by a command via a touchscreen, button or other user interface element. At this point, the cuff still deflated and the pump is not running. Subsequently, an integral over the air flow (IAF) is set to zero in step 102, which is done before the inflation of the cuff 10 begins. Then, the NIBP measurement (step 103) is performed including steps of inflating the cuff 10 and measuring the oscillation amplitudes, which can generally be measured during inflation or during deflation (or both, if the measurement during inflation did not yield a conclusive result). At the end of the measurement the cuff 10 is deflated (step 104). In parallel, the air flow into the cuff 10 and out of the cuff 10 is integrated (step 105).

In step 106 it is checked if the IAF exceeds an allowed residual value. If there is a significant difference between the two amounts (i.e. if the IAF is larger than the residual value), this may be interpreted as deflation failure, and a failure signal, e.g. an alarm, may be issued (step 107), which e.g. instructs the user to remove the cuff 10 from the patient and optionally check the cuff 10 for possible blockages. Otherwise (i.e. if the IAF is not larger than the residual value), this may be interpreted as a correct deflation and the measurement is ended (step 108).

The residual value may be set in advance, e.g. as a general value, as value dependent on a patient category or even as patient-individual value. For instance, the caregiver may initially set the residual value using the user interface 26. In other embodiments, the residual value may be automatically set, e.g. selected from a table or computed based on an algorithm. One or more of the following parameters and factors may be used for setting the residual value:
- size or maximum gas volume that can be pumped into the cuff;
- one or more features of the subject, the one or more features including age, gender, height, weight, and health status;
- maximum gas volume pumped into the cuff during blood pressure measurement;
- maximum cuff pressure during blood pressure measurement;
- a constant threshold; and
- a fraction of the air volume pumped into the cuff.

As the cuffs used for the measurement are generally not completely elastic, not all of the air pumped into the cuff during the measurement will exit the cuff when it is deflated. In order to avoid false alarms, some residual air volume is thus allowed to remain in the cuff. The amount of this residual air volume may thus be a function of the detected cuff size (cuffs range from tiny for neonatal patient to fairly large for measurements on the patients thigh), the patient category, the total air volume pumped into the cuff during the measurement, and/or the maximum cuff pressure attained during the measurement.

The failure detection method 100 may particularly be implemented into an automated NiBP monitor, or as an external device that is inserted into the pneumatic path. The external version may work with less information about the measurement process, but it can check that the cuff 10 reaches a fully deflated state once every few minutes. It can also attempt to infer when a measurement is ongoing and when the measurement ends from the air flow in in the pneumatic path in order to provide more precise alarms.

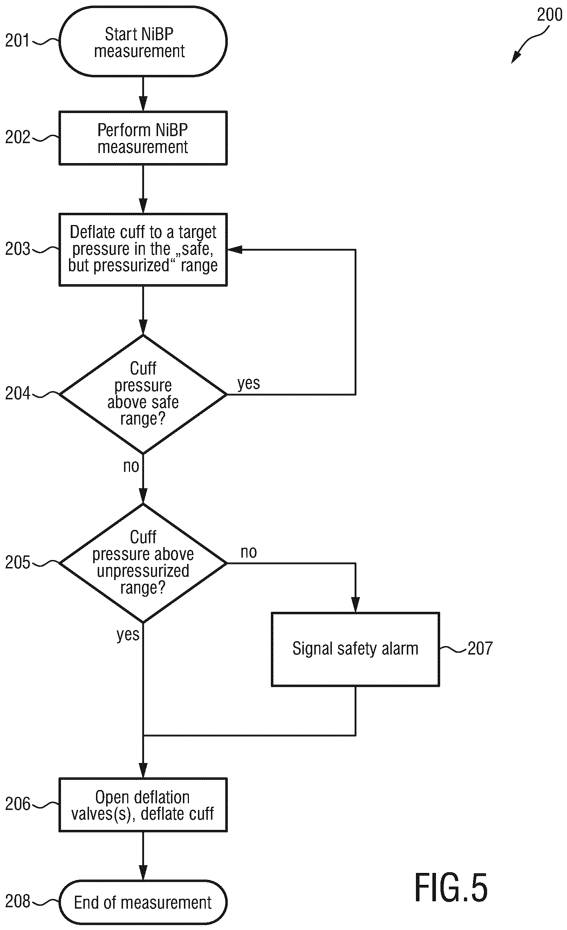

Fig. 5 shows a flow chart of a second embodiment of a failure detection method 200 according to the present invention, which may be carried out by a failure detection device 25 in a blood pressure monitoring device 20 shown in Fig. 1 since it does not require any additional sensors in addition to the elements of a conventional NIBP monitor. It works by using the pressure sensor 23 that is also used to measure cuff pressure and oscillations during the measurement.

After starting the NIBP measurement in step 201 the NIBP measurement (step 202) is performed, at the end of which the cuff 10 is deflated (step 203) by opening the valve 22. to a target pressure in a safe range (i.e. below a safety cuff pressure (or safety threshold) above which the pressure is harmful to the patient but above ambient pressure or an unpressurized state of the cuff). Hereby, the deflation is intermittently (e.g. periodically) stopped to measure the remaining pressure in the cuff (generally, the cuff pressure cannot be measured when the valves are open as the pressure sensor is close to the valves since in this case ambient pressure instead of cuff pressure would be measured).

In step 204 it is checked if the cuff pressure is above the safe range. If yes, the deflation process is initiated again (step 203). If the measured cuff pressure is below the potentially harmful safety cuff pressure, it is further checked (step 205) if the measured cuff pressure is still higher than ambient pressure, i.e. above the unpressurized range, representing a second pressure threshold. If it is still higher than ambient pressure, cuff deflation is considered successful and deflation is continued (step 206) until the measurement process is ended (step 208). If at any point during the measurement or the deflation process the cuff pressure drops from the potentially harmful range or safe range to the ambient pressure value, this may be interpreted as deflation failure, and a failure signal, e.g. an alarm, may be issued (step 207).

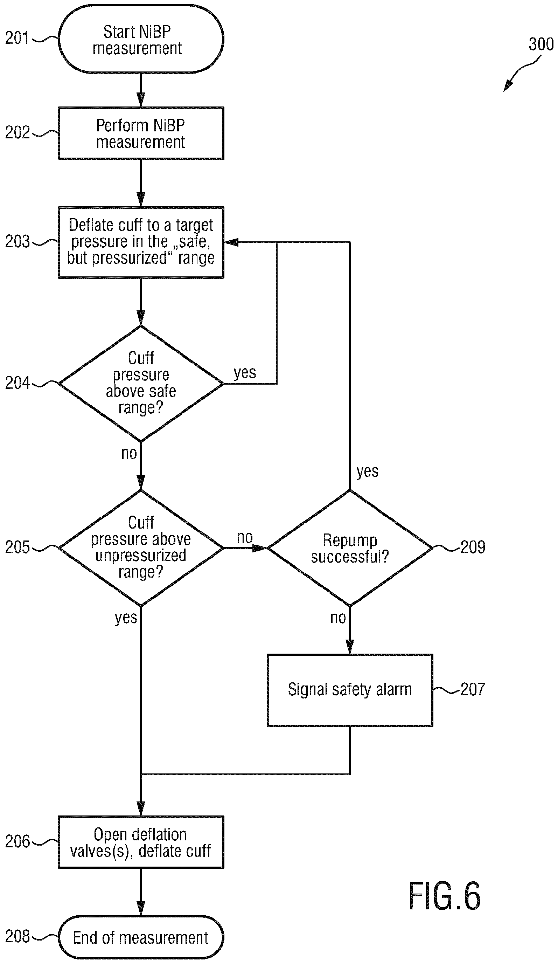

Fig. 6 shows a flow chart of a third embodiment of a failure detection method 300 according to the present invention, which may be carried out by a failure detection device 25 in a blood pressure monitoring device 20 shown in Fig. 1 as well. The method 300 is rather similar to the method 200 shown in Fig. 5 and the same steps are indicated by the same reference numbers. In an additional step 209 the cuff 10 is re-inflated if an intermittently measured remaining cuff pressure value (as checked in steps 204 and 205) is below the safety cuff pressure and below the second pressure threshold (representing ambient pressure or unpressurized state). Further, it is interpreted as deflation failure if the re-inflation was not successful so that a failure signal may be issued (step 207).

Regardless of the method being used, it may also be checked for events that indicate that the pneumatic path has become blocked during the measurement. Such events may include a sudden large jump in pressure when the pump is running, as air is no longer entering the cuff but gets compressed inside the much smaller volume of the air hose, and a drop from any pressure value in the possibly harmful range to the ambient pressure value after the valve has been opened and closed to perform a deflation step. As the cuff pressure can be estimated after a deflation step, any large deviation from the predicted new pressure value can be taken as a sign that the pneumatic path is compromised either by leakage or a blockage.

Thus, in an embodiment a deflation failure may be detected by detecting a sudden large change of the cuff pressure during inflation, in particular a change of the cuff pressure exceeding a change threshold within a predetermined period, and/or by detecting a deviation above a deviation threshold from a predicted cuff pressure that is predicted based on the obtained pressure signal and/or the obtained gas flow signal.

According to the present invention a failure to fully deflate/exhaust the cuff after the measurement is concluded is quickly and reliably detected so that a corresponding safety alarm can be issued since the failure cannot be remedied autonomously by the blood pressure monitor, and prolonged exposure to even relatively low cuff pressures can cause injury to the patient, so the failure condition requires more attention than just a technical alarm.

The embodiments of the devices and methods using an existing pressure sensor may generally be implemented without any additional hardware compared to conventional devices. The embodiments of the devices and methods using the flow meter do not require changes to the deflation process and may less likely generate false alarms and may have a shorter total measurement time since no interruptions of the deflation process are needed to take pressure measurements.

The present invention thus presents devices and methods for timely signalling an alarm if the cuff wrapped around a patient's extremity fails to deflate after a non-invasive blood pressure measurement. Failure to deflate to cuff can lead to injury in cases where the patient is unconscious or otherwise helpless. This risk can be mitigated by ascertaining that deflation below potentially harmful cuff pressure did occur after each measurement.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Failure detection device (25) for a blood pressure monitoring device (20, 20'), the failure detection device comprising:
- a data input (251) configured to obtain a pressure signal and/or a gas flow signal, the pressure signal indicating a cuff pressure of a cuff (10) that is configured to be mounted to a subject's body part and to be inflated for performing a blood pressure measurement and the gas flow signal indicating gas flow entering the cuff during inflation and leaving the cuff during deflation;
- a signaling output (252) configured to output a failure signal; and
- a processor (253) configured to detect, based on the obtained pressure signal and/or the obtained gas flow signal, a deflation failure indicating a failure to deflate the cuff after a blood pressure measurement below a safety cuff pressure and to control the signaling output (252) to output a failure signal indicating a deflation failure in case a deflation failure is detected.

2. Failure detection device as claimed in claim 1,
wherein the processor (253) is configured to
- detect a deflation failure based on the obtained cuff pressure signal,
- control a valve (22) that is configured to deflate the cuff to intermittently deflate the cuff after the blood pressure measurement,
- control a pressure sensor (23) that is configured to measure the cuff pressure to measure the remaining cuff pressure in between the periods of deflation, and
- detect a deflation failure based on the intermittently measured remaining cuff pressure values.

3. Failure detection device as claimed in claim 2,
wherein the processor (253) is configured to check, for the intermittently measured remaining cuff pressure values, if the remaining cuff pressure is below the safety cuff pressure.

4. Failure detection device as claimed in claim 3,
wherein the processor (253) is configured to check if an intermittently measured remaining cuff pressure value is below the safety cuff pressure and below a second pressure threshold corresponding to ambient pressure or unpressurized state of the cuff and to detect it as deflation failure if the previous intermittently measured pressure value was above the safety cuff pressure and the current intermittently measured remaining cuff pressure value is below the safety cuff pressure and below the second pressure threshold.

5. Failure detection device as claimed in claim 3,
wherein the processor (253) is configured to
- check if an intermittently measured remaining cuff pressure value is below the safety cuff pressure and below a second pressure threshold corresponding to ambient pressure or unpressurized state of the cuff,
- control a pressure generating unit to re-inflate the cuff (10) if an intermittently measured remaining cuff pressure value is below the safety cuff pressure and below the second pressure threshold, and
- detect it as deflation failure if the re-inflation was not successful.

6. Failure detection device as claimed in any one of the preceding claims, wherein the processor (253) is configured to detect a deflation failure based on the obtained gas flow signal, wherein the obtained gas flow signal indicates a first measured amount of gas entering the cuff during inflation and a second measured amount of gas leaving the cuff during deflation.

7. Failure detection device as claimed in claim 6,
wherein the processor (253) is configured to obtain a difference value by subtracting the second amount from the first amount and to control the signaling output (252) to output a failure signal indicating a deflation failure if the difference value exceeds a residual value.

8. Failure detection device as claimed in claim 7,
wherein the processor (253) is configured to set the residual value based on one or more of:
- size or maximum gas volume that can be pumped into the cuff;
- one or more features of the subject, the one or more features including age, gender, height, weight, and health status;
- maximum gas volume pumped into the cuff during blood pressure measurement;
- maximum cuff pressure during blood pressure measurement
- a constant threshold; and
- a fraction of the air volume pumped into the cuff.

9. Failure detection device as claimed in any one of the preceding claims, wherein the signaling output (252) is configured to output a failure signal indicating one or more of an instruction to remove the cuff from the subject's body part, an urgency of the deflation failure, and a strength of the deflation failure.

10. Failure detection device as claimed in any one of the preceding claims, wherein the processor (253) is configured to detect a deflation failure by detecting a sudden large change of the cuff pressure during inflation, in particular a change of the cuff pressure exceeding a change threshold within a predetermined period, and/or by detecting a deviation above a deviation threshold from a predicted cuff pressure that is predicted based on the obtained pressure signal and/or the obtained gas flow signal.

11. Blood pressure monitoring device (20, 20') comprising:
- a pressure generating unit (21) configured to inflate a cuff (10) that is configured to be mounted to a subject's body part;
- a valve (22) configured to deflate the cuff;
- a pressure sensor (23) configured to measure the cuff pressure;
- a processor (24) configured to control the pressure generating unit (21) and the valve (22) and to determine the subject's blood pressure based on the measured cuff pressure; and
- a failure detection device (25) as claimed in any one of claims 1 to 11.

12. Blood pressure monitoring device as claimed in claim 11,
further comprising a flow meter (27) configured to measure gas flow entering the cuff during inflation and leaving the cuff during deflation.

13. Blood pressure monitoring system (1, 1') comprising:
- a cuff (10) configured to be mounted to a subject's body part; and
- a blood pressure monitoring device (25) as claimed in any one of claims 11 to 12, the blood pressure monitoring device being configured to control the cuff and non-invasively measure the subject's blood pressure.

14. Failure detection method for a blood pressure monitoring device (20, 20'), the failure detection method comprising:
- obtaining a pressure signal and/or a gas flow signal, the pressure signal indicating a cuff pressure of a cuff (10) that is configured to be mounted to a subject's body part and to be inflated for performing a blood pressure measurement and the gas flow signal indicating gas flow entering the cuff during inflation and leaving the cuff during deflation;
- detecting, based on the obtained pressure signal and/or the obtained gas flow signal, a deflation failure indicating a failure to deflate the cuff after a blood pressure measurement below a safety cuff pressure; and
- control a signaling output (252) to output a failure signal indicating a deflation failure in case a deflation failure is detected.

15. Computer program comprising program code means for causing a computer to carry out the steps of the method as claimed in claim 14 when said computer program is carried out on the computer.
